# EUROPEAN PATENT APPLICATION

(11) **EP 4 810 067 A1**
(43) Date of publication of application: **23.09.2026**
(21) Application number: 24890271.0
(22) Date of filing: 30.08.2024
(51) Int. Cl.: A61K 47/69, A61K 47/24, A61K 47/14, A61K 38/16

(54) **BIOTIN OR AVIDIN-LABELED FAT BODY, PREPARATION METHOD THEREFOR, AND NANO-CARRIER SYSTEM**

(30) Priority: 14.11.2023 CN 202311512319
(71) Applicant: WeCareLife Biotech Co., Ltd., Beijing 102629 (CN)
(72) Inventor: CAO, Zhen, Beijing 102629 (CN); PAN, Bin, Beijing 100101 (CN); ZHANG, Gaoxin, Beijing 100101 (CN); ZHI, Zelun, Beijing 102629 (CN); LI, Zemin, Beijing 100101 (CN)
(74) Representative: Eisenführ Speiser
(86) International application number: PCT/CN2024/115749
(87) International publication number: WO 2025/102910

(57) **Abstract**

A biotin or avidin-labeled fat body, a preparation method therefor, and a nano-carrier system. A specific amount of bio-phospholipid or avidin-phospholipid is used to prepare a biotin or avidin labeled fat body. The prepared fat body has the advantages of high purity, small particle size, good uniformity and high stability. A nano-carrier system is constructed on the basis of the biotin-avidin system, and the nano-carrier system comprises the biotin-labeled fat body and an avidin-modified target component. By utilizing the system, the fat body can carry any substance of interest, for example protein drugs such as virus recombinant proteins, and antibodies, nucleic acid drugs or small molecule drugs, thereby being used for vaccines and the treatment of diseases such as cancers, infectious diseases and metabolic diseases.

## Description

This application claims the priority of Chinese Patent Application No. 202311512319.8, filed with the China National Intellectual Property Administration on November 14, 2023, and entitled "BIOTIN OR AVIDIN-LABELED ADIPOSOME, PREPARATION METHOD THEREFOR, AND NANO-CARRIER SYSTEM", which is hereby incorporated by reference in its entirety.

### FIELD

The present disclosure relates to the field of biomedicine, in particular to a biotin- or avidin-labeled adiposome, a production method thereof, and a nano-carrier system.

### BACKGROUND

Nanoparticles refer to particles having a particle size ranging from 1 to 1000 nm, and are novel drug carriers that have unique advantages in improving delivery efficiency, reducing side effects, and enhancing targeting capability of a drug. As a novel nanoparticle, adiposome is a nanosphere composed of a neutral lipid core encapsulated by a phospholipid monolayer membrane, similar in structure to naturally occurring lipid droplets and lipoproteins, and has significant application value in the biomedical field. However, general methods and strategies for enabling adiposomes to possess specific targeting capability and multifunctionality remain to be addressed.

Biotin-avidin technology is a non-covalent interaction, wherein the interaction of biotin and avidin has extremely high affinity and specificity, with a K_{d} of 10⁻¹⁴ to 10⁻¹⁵. Currently, biotin-avidin technology is widely used in fields such as affinity purification of proteins, cell labeling and tracking, immunoassay and diagnostics.

Currently, there have been no reports linking adiposomes with the biotin-avidin system and applying them to targeted therapy of diseases using nano-delivery systems. The present disclosure constructs a general adiposome linker technology based on biotin-avidin, and the prepared nanocarrier system enables adiposomes to carry any cargo of interest, such as carrying viral recombinant proteins for vaccine development, carrying antibodies for targeted drug delivery, or carrying nucleic acid-based drugs, thereby is capable of being used for vaccine production and the treatment of diseases including cancers, infectious diseases, and metabolic diseases, and having extremely significant medical application value.

### SUMMARY

The present disclosure provides a biotin- or avidin-labeled adiposome, a production method thereof, and a nanocarrier system. The nanocarrier is a biotin- or avidin-labeled adiposome having high purity, small particle size, good uniformity, and high stability, and is capable of carrying any cargo of interest, having broad medical application value.

The present disclosure provides a method for producing a biotin- or avidin-labeled adiposome, comprising:
mixing biotin- or avidin-labeled phospholipid, unlabeled phospholipid, and neutral lipid in a solvent, and performing reaction to obtain the biotin- or avidin-labeled adiposome..

In the present disclosure, when producing avidin- or biotin-labeled adiposomes, adiposomes may be produced first, and then labeled with avidin or biotin. Labeling may be performed by methods commonly used in the art according to the groups located on the adiposome surface, such as chemical coupling methods and incubation methods.

In the present disclosure, the mass percentage of the amount of biotin- or avidin-labeled phospholipids fed relative to the total amount of phospholipids is X, and 0<X≤100%. Research results show that when 0<X<40%, labeled adiposomes having high purity and good uniformity can be obtained without additional purification steps after the reaction is completed. When 10%≤X≤20%, the prepared labeled adiposomes can be better used to recruit cargo proteins of interest (e.g., GFP-avidin), and the formed green ring-like structures are more uniform, and the morphology of the adiposomes is more complete. When the amount of Bio-PE added is greater than or equal to 40%, i.e., 40%≤X≤100%, the produced labeled adiposomes have more membrane impurities and a polydispersity index (PDI ) greater than 0.2, but the purity and uniformity of the adiposomes can be improved by adding an additional purification step after the reaction is completed, thereby obtaining adiposomes having high purity and good uniformity. Furthermore, research in the present disclosure finds that adiposomes of different particle sizes can be obtained by controlling X to meet different application needs; with the increase of X, the particle size of the formed adiposomes gradually decreases, with the average particle size decreasing from about 120 nm to about 70 nm. In some embodiments, 0<X<40%; specifically, X may be a value range or a specific value selected from the group consisting of 0<X<1.25%, 1.25%≤X≤5%, 5%<X<10%, 10%≤X≤20%, 20%<X<40%, 1.25%, 2.5%, 5%, 7.5%, 10%, 12.5%, 15%, 17.5%, 20%, 22.5%, 25%, 27.5%, 30%, 32.5%, 35%, 37.5%, 38%, and 39%. In some other embodiments, 40%≤X≤100%; specifically, 40%≤X<60%, 60%≤X<80%, or 80%≤X≤100%, or X is selected from the group consisting of 40%, 45%, 50%, 55%, 60%, 65%, 70%, 75%, 80%, 85%, 90%, 95%, and 100%.

In the present disclosure, the purification comprises:
first centrifuging at 15000 g to 2300 g for 5 to 10 min, removing a precipitate, and resuspending; then centrifuging at 500 g to 1500 g for 5 to 10 min, removing an upper white band, and resuspending; repeating this step 3 times.

In some specific embodiments, the purification comprises: first centrifuging at 21000 g for 6 min, removing the precipitate, resuspending, then centrifuging at 1000 g for 6 min, removing the upper white band, and resuspending; repeating this step 3 times.

In the present disclosure, a solution used for the resuspending is phosphate buffer, 15 mM to 25 mM HEPES buffer, NaCl solution, KCl solution, or MgCl₂ solution; in some specific embodiments, the solution used for the resuspending is phosphate buffer.

PDI refers to polydispersity index, which is an indicator used to measure the uniformity of nanoparticles; the smaller the PDI, the more uniform the particle size distribution. When PDI is less than 0.2, the uniformity is generally considered good, whereas when PDI is greater than 0.2, the uniformity of nanoparticles is relatively poor. Results show that when the amount of biotin-labeled phospholipid (Bio-phospholipid) added is 1.25% to 20%, the average particle size gradually decreases, and the PDI is all less than 0.2; when the amount of biotin-labeled phosphatidylethanolamine (Bio-PE) added is 20%, the average particle size reaches 75.6 nm, and the PDI is less than 0.2; whereas when Bio-phospholipid is added to 40%, optical microscopy shows many membrane impurities of non-adiposome-like structure, and the PDI is all greater than 0.2, significantly exceeding that of adiposomes labeled with 20% Bio-phospholipid, but after additional purification, the purity and uniformity are improved, with PDI below 0.2. This indicates that the amount of Bio-phospholipid added is one of the important factors in forming biotin- or avidin-labeled adiposomes, and when the amount added reaches or exceeds 40%, additional purification steps are required to obtain adiposomes having high purity and good uniformity.

The present disclosure has no special requirements for the method of mixing the labeled and unlabeled phospholipids; they may be dissolved in chloroform separately and then mixed, or mixed first and then dissolved in chloroform. In some embodiments of the present disclosure, they are first dissolved in chloroform separately and then mixed; specifically, the biotin- or avidin-labeled phospholipids and the unlabeled phospholipids are dissolved in chloroform separately to a final concentration of 25 mg/mL, and then the two solutions are mixed at a volume ratio of (0 to 80):(0 to 80) such that the mass percentage of the biotin- or avidin-labeled phospholipids relative to the total amount of phospholipids is 0 to 100%.

In the present disclosure, the neutral lipid is at least one selected from the group consisting of triglyceride, cholesteryl ester, retinyl ester, ether ester, sterol ester, and polyhydroxyalkanoate; in a specific embodiment, the neutral lipid is triglyceride; the phospholipid is at least one selected from the group consisting of phosphatidylcholine (PC), phosphatidic acid (PA), phosphatidylserine (PS), phosphatidylethanolamine (PE), and phosphatidylinositol (PI). In a specific embodiment, the phospholipid is dioleyl phosphatidylcholine (DOPC) and/or PE. In some specific embodiments, the unlabeled phospholipid is unlabeled DOPC, and the biotin-labeled phospholipid is biotin-labeled PE; a mass ratio of the unlabeled phospholipid, the biotin-labeled phospholipid, and the neutral lipid is (0 to 2):(0 to 2):(3 to 10); in some embodiments, the mass ratio is (0 to 2):(0 to 2):3, (0 to 2):(0 to 2):4, (0 to 2):(0 to 2):5, (0 to 2):(0 to 2):6, (0 to 2):(0 to 2):7, (0 to 2):(0 to 2):8, (0 to 2):(0 to 2):9, or (0 to 2):(0 to 2):10, preferably (0 to 2):(0 to 2):5; further, the mass ratio is (1.975 to 2):(0 to 0.025):5, (1.2 to 1.975):(0.025 to 0.8):5, (1.6 to 1.975):(0.025 to 0.4):5, (1.6 to 1.8):(0.2 to 0.4):0.5, (1.2 to 1.6):(0.4 to 0.6):5, (0 to 1.2):(0.8 to 2):5, (0.4 to 1.2):(0.8 to 1.6):5, or (0 to 0.4):(1.6 to 2):5; specifically, the mass ratio may be 1.975:0.025:5, 1.9:0.1:5, 1.8:0.2:5, 1.7:0.3:5, 1.6:0.4:5, 1.5:0.5:5, 1.4:0.6:5, 1.3:0.7:5, 1.2:0.8:5, 1.1:0.9:5, 1:1:5, 0.9:1.1:5, 0.8:1.2:5, 0.7:1.3:5, 0.6:1.4:5, 0.5:1.5:5, 0.4:1.6:5, 0.3:1.7:5, 0.2:1.8:5, 0.1:1.9:5, or 0:2:5, preferably 1.8:0.2:5 or 1.6:0.4:5.

In the present disclosure, the solvent is at least one selected from the group consisting of phosphate buffer, HEPES buffer, sucrose solution, NaCl solution, KCl solution, and MgCl₂ solution; in some specific embodiments of the present disclosure, the solvent is phosphate buffer.

In the present disclosure, the mixing includes vortex mixing, ultrasonic mixing, stirring mixing, or high-pressure homogenization mixing. In some embodiments, the mixing is vortex mixing; in some specific embodiments, the vortexing is performed by: vortexing at an intensity of 3000 to 4000 rpm, vortexing for 8 to 15 s and pausing for 5 to 15 s each time, for a total duration of 3 to 7 min. In a specific embodiment, the vortexing is performed by: vortexing at an intensity of 4000 rpm, vortexing for 10 s and pausing for 10 s each time, for a total duration of 5 min.

In the present disclosure, after the reaction is completed, the method further comprises steps of centrifuging and collecting a liquid phase. In some embodiments, the centrifuging and collecting of the liquid phase specifically comprise:
1) subjecting the vortexed solution to a first centrifugation at 100 to 1000 g for 3 to 7 min, and removing the upper white band; performing a second centrifugation at 18000 to 22000 g for 3 to 7 min, removing a precipitate, and collecting a liquid-phase emulsion;
2) resuspending the liquid-phase emulsion, performing a third centrifugation at 100 to 1000 g for 3 to 7 min, removing an upper white band, and collecting a liquid-phase emulsion to obtain a biotin-labeled adiposome.

In some specific embodiments, in the above-mentioned step 1), the first centrifugation is performed at 1000 g for 5 min; and the second centrifugation is performed at 20000 g for 5 min; and in the above-mentioned step 2), the third centrifugation is performed at 1000 g for 5 min.

The present disclosure further provides a biotin- or avidin-labeled adiposome produced by the method of the present disclosure.

The present disclosure provides a biotin- or avidin-labeled adiposome, wherein a phospholipid monolayer of the adiposome comprises a biotin- or avidin-labeled phospholipid; and in the phospholipid monolayer of the adiposome, an average percentage of the content of the biotin- or avidin-labeled phospholipid is Y, and 0 < Y ≤ 100%, preferably, 5 ≤ Y ≤ 100%.

In the present disclosure, the lipid composition in the adiposomes containing different amounts of biotin is analyzed and detected by thin-layer chromatography (TLC). The grayscale value of each sample on the silica gel plate is analyzed by software Image J to calculate the content percentage Y of biotin-labeled phospholipid in the phospholipid monolayer of the adiposome. In some embodiments, Y is selected from the group consisting of 5% to 20%, 10% to 20%, 15% to 30%, 20% to 40%, 35% to 65%, and 60% to 100%, or is 5% to 10%, 10% to 15%, 15% to 20%, 20% to 25%, 25% to 30%, 35% to 40%, 40% to 45%, 45% to 50%, 50% to 55%, 55% to 60%, 60% to 65%, 65% to 70%, 75% to 80%, 85% to 90%, or 95% to 100%; specifically, Y may be 7.24%, 14.38%, 18.05%, 25.71%, 38.05%, 62.2%, or 100%.

Experiments show that the biotin- or avidin-labeled adiposomes produced by the present disclosure have an average particle size of 60 to 150 nm and a PDI less than 0.2. After storage at 4°C for 1 day, 9 days, 15 days, and 22 days, the change in particle size and PDI was measured by dynamic light scattering, and the results show that the particle size of adiposomes containing different amounts of biotin or avidin remained unchanged, and the PDI also remained essentially unchanged. The above results indicate that the biotin- or avidin-labeled adiposomes prepared by the present disclosure have small particle size, good uniformity, and high stability. Further, the average particle size is 65 to 120 nm, or 60 nm to 65 nm, 65 nm to 70 nm, 70 nm to 75 nm, 75 nm to 80 nm, 80 nm to 85 nm, 85 nm to 90 nm, 90 nm to 95 nm, 95 nm to 100 nm, 100 nm to 105 nm, 105 nm to 110 nm, 110 nm to 115 nm, 115 nm to 120 nm, 120 nm to 130 nm, 130 nm to 140 nm, or 140 nm to 150 nm, and may specifically be 117.3 nm, 110.0 nm, 83.17 nm, 78.1 nm, 75.6 nm, 106.77 nm, 102.57 nm, 83.83 nm, 75.07 nm, 71.1 nm, 74.53 nm, 74.27 nm, or 68.13 nm.

The present disclosure further provides use of the biotin- or avidin-labeled adiposome obtained by the above-mentioned production method as a nanocarrier in the manufacture of nanomedicines.

The present disclosure further provides a nanocarrier system, comprising a biotin-labeled adiposome and an avidin-labeled cargo of interest;
or comprising an avidin-labeled adiposome and a biotin-labeled cargo of interest;
the adiposome and the cargo of interest are connected non-covalently via biotin and avidin.

In some embodiments, the biotin-labeled adiposome is produced by the production method of the present disclosure, or is the biotin-labeled adiposome of the present disclosure; the avidin-labeled adiposome is produced by the production method of the present disclosure, or is the avidin-labeled adiposome of the present disclosure.

In a specific example of the present disclosure, the ability of adiposomes produced with different amounts of Bio-PE (0%, 1.25%, 5%, 10%, and 20%, respectively) to recruit the target protein GFP-avidin was examined. The results show that adiposomes prepared with 10%-20% Bio-PE were better for recruiting the target protein GFP-avidin, where the formed green ring-shaped structures were more uniform and the morphology of the adiposomes was more complete. In contrast, in adiposomes prepared with other amounts of Bio-PE (Bio-PE content of 1.25%-5%), GFP-avidin mostly appeared as green dot-shaped structures, the morphology of the adiposomes were affected, and the adiposomes aggregated. This indicates that the content of biotin in the biotin-labeled adiposomes affects the amount and shape of the recruited protein, as well as the morphology and stability of the adiposomes. Among them, adiposomes produced with 10% to 20% Bio-PE were more favorable for recruiting avidin-tagged target proteins, and further, biotin-labeled adiposomes produced with 20% Bio-PE had the strongest recruitment ability.

In some embodiments, the cargo of interest is a pharmaceutically active ingredient, including a peptide, an antibody, a nucleic acid, or a small molecule. The nucleic acid is preferably DNA or RNA, and further preferably one or more of mRNA, RNAi, siRNA, shRNA, miRNA, sgRNA, and crRNA.

In some embodiments, the cargo of interest is a marker protein, including GFP protein, mCherry protein, Luciferase protein, and/or horseradish peroxidase.

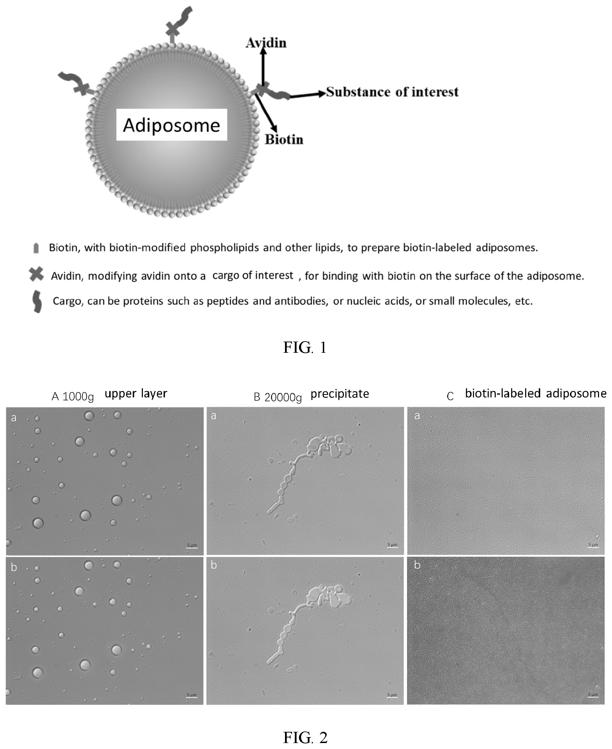

As shown in FIG. 1, on one hand, the nanocarrier system provided by the present disclosure uses biotin-labeled adiposomes produced from phospholipids or other polar lipids modified with biotin, and on the other hand, the nanocarrier system provided by the present disclosure uses avidin-tagged recombinant proteins obtained via expression and purification, or nucleic acids, small molecules, and other substances tagged with avidin obtained via chemical modification, wherein the avidin-tagged target components bind to biotin on the adiposome surface in a non-covalent manner. The nanocarrier system provided by the present disclosure is constructed based on biotin-avidin to enable adiposomes to carry any cargo of interest, such as carrying viral recombinant proteins for vaccine development, or carrying antibodies for targeted drug delivery, or delivering nucleic acids, etc., thereby is capable of being used for the treatment of diseases such as cancers, infectious diseases, and metabolic diseases, and having extremely significant medical application value.

In some embodiments, the cargo of interest is a marker protein, including GFP protein, mCherry protein, Luciferase protein, or horseradish peroxidase (HRP).

In a specific example, the present disclosure constructed an avidin-tagged recombinant protein GFP-avidin, with an amino acid sequence as set forth in SEQ ID NO: 1, and a nucleic acid sequence as set forth in SEQ ID NO: 2. After the Bio-PE produced by the present disclosure and the avidin-tagged recombinant protein GFP-avidin were vortexed, incubated, and centrifuged, the liquid-phase emulsion was resuspended and identified by confocal laser scanning microscopy and silver staining analysis. The results show that the recombinant protein GFP-avidin was capable of binding to the biotin-labeled adiposomes. Among them, when the recruited protein was GFP protein, the ring-shaped structures formed by biotin-labeled adiposomes produced with 10% to 20% Bio-PE (when the mass percentage of Bio-PE to total phospholipids was 10% to 20%) were more uniform, and the morphology of the adiposomes was more complete, indicating that the adiposomes had the strongest ability to recruit the target protein GFP-avidin.

The present disclosure provides a nanocarrier, i.e. a biotin- or avidin-labeled adiposome, which is capable of carrying any cargo of interest through the biotin-avidin system. Experiments showed that the biotin-labeled adiposomes produced by the present disclosure had an average particle size of 60 to 150 nm and a PDI less than 0.2. After storage at 4°C for 1 day, 9 days, 15 days, and 22 days, the change in particle size and PDI was measured by dynamic light scattering, and the results show that the particle size of adiposomes containing different amounts of biotin remained unchanged, and the PDI also remained essentially unchanged. The above results indicate that the biotin-labeled adiposomes produced by the present disclosure have small average particle size, good uniformity, and high stability.

### BRIEF DESCRIPTION OF DRAWINGS

FIG. 1 shows a schematic structural diagram of a nanocarrier system consisting of adiposome, biotin-avidin linker, and cargo (such as pharmaceutically active ingredients);
FIG. 2 shows optical microscopy images during the production of biotin-labeled adiposomes; wherein A shows the optical microscopy result of the upper white band after centrifugation at 1000 g for 5 min in step 3; B shows the optical microscopy result of the pellet after centrifugation at 20000 g for 5 min in step 4; C shows the optical microscopy result of the final biotin-labeled adiposomes in step 5; a shows the differential interference contrast (DIC) result, b shows the result of adiposomes stained with the neutral lipid-specific dye LipidTox Red, and the scale bar is 5 µm;
FIG. 3 shows the particle size and PDI of biotin labeled-adiposomes at different storage times; adiposomes produced with 0%, 1.25%, 5%, 10%, and 20% Bio-PE were stored at 4°C for 1 day (blue), 9 days (red), 15 days (green), and 22 days (purple), respectively, and then the changes in particle size and PDI were detected;
FIG. 4 shows the purity and uniformity results of adiposomes with different Bio-PE contents, wherein A shows adiposomes produced with 20% Bio-PE, B shows adiposomes produced with 40% Bio-PE, and C shows the PDI of adiposomes;
FIG. 5 shows the average particle size (A) and PDI (B) of adiposomes labeled with different contents of biotin by inputting Bio-PE at a mass ratio from 0% to 100% through the optimized purification method;
FIG. 6 shows TLC analysis for detecting the lipid composition of biotin-labeled adiposomes; lanes 1 to 5 are adiposomes produced with 0%, 1.25%, 5%, 10%, and 20% Bio-PE, respectively;
FIG. 7 shows lipid composition analysis of 8 adiposome samples in three independent experiments (A) and the content percentage of biotin-labeled phospholipids in the phospholipid monolayer of adiposome (B);
FIG. 8 shows the construction of avidin-tagged recombinant protein GFP-avidin; the upper part shows the Coomassie staining results of each sample during the purification process, and the lower part shows the WB results;

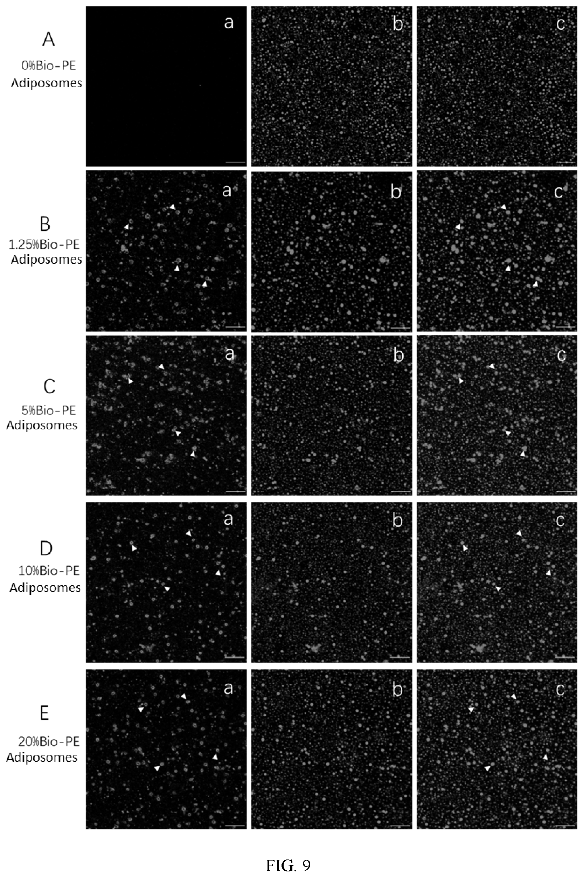

FIG. 9 shows the recruitment of recombinant protein GFP-avidin by biotin-labeled adiposomes; A to E show adiposomes produced with 0%, 1.25%, 5%, 10%, and 20% Bio-PE, respectively, which were incubated with avidin-tagged recombinant protein GFP-avidin, purified by centrifugation, and observed whether there were fluorescent signals on the adiposome surface; wherein a shows the green signal of recombinant protein GFP-avidin, b shows adiposomes stained with the neutral lipid-specific dye LipidTox Red, and c is the merged image of channels a and b; white arrows indicate fluorescent signals on the spherical surface, indicating that adiposomes in this group can recruit GFP-avidin signals, and the scale bar is 5 µm;

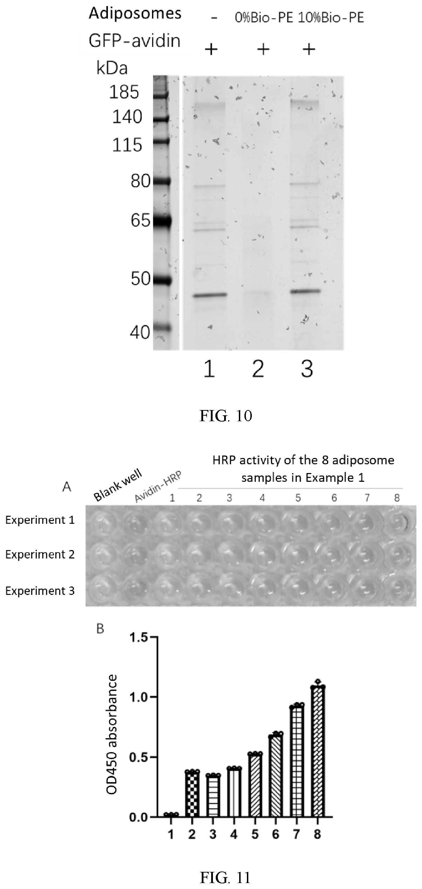

FIG. 10 shows the recruitment of recombinant protein GFP-avidin by biotin-labeled adiposomes; adiposomes produced with 0% and 10% Bio-PE were incubated with avidin-tagged recombinant protein GFP-avidin, respectively, and purified by centrifugation, and silver staining analysis was performed to identify whether there was recombinant protein GFP-avidin on the adiposome surface. Lane 1 shows only recombinant protein GFP-avidin added without adiposomes; lane 2 shows recombinant protein GFP-avidin added with adiposomes unlabeled with biotin; lane 3 shows recombinant protein GFP-avidin added with biotin-labeled adiposomes;
FIG. 11 shows that biotin-labeled adiposomes carry the protein horseradish peroxidase (HRP) and have biological activity; A shows the HRP activity in adiposomes of 8 samples produced by the optimized method of Example 1; B shows the average absorbance at 450 nm of each well;

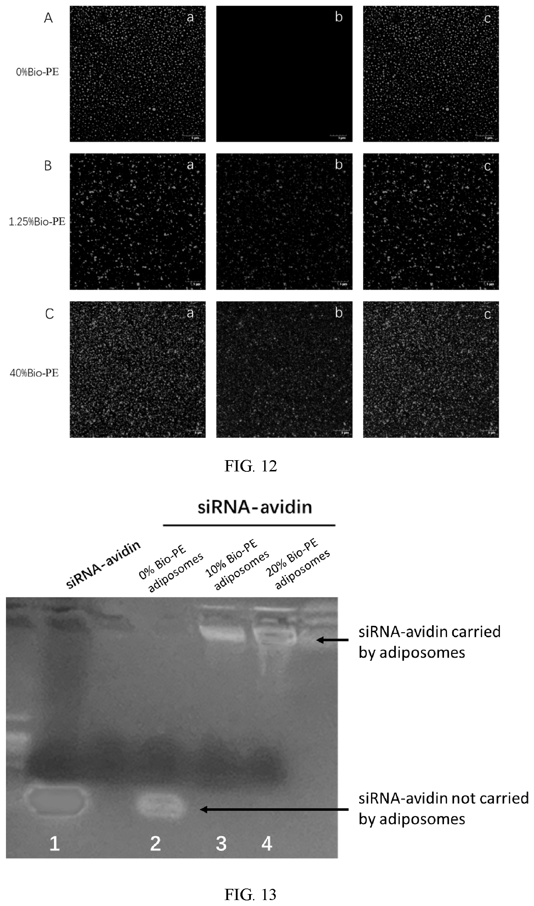

FIG. 12 shows that Biotin-labeled adiposomes carry the small-molecule fluorescein isothiocyanate (FITC); A shows the result after incubation of adiposomes produced with 0% Bio-PE with fluorescein isothiocyanate FITC; B shows the result after incubation of adiposomes prepared with 1.25% Bio-PE with fluorescein isothiocyanate FITC; C shows the result after incubation of adiposomes produced with 40% Bio-PE with fluorescein isothiocyanate FITC; a shows adiposomes stained with the neutral lipid-specific dye LipidTox Red, b shows avidin-labeled fluorescein isothiocyanate (FITC), and c is the merged image of a and b, with a scale bar of 5 µm;
FIG. 13 shows that Biotin-labeled adiposomes carry nucleic acid substances; adiposomes produced with 0%, 10%, and 20% Bio-PE were incubated with avidin-tagged small nucleic acid siRNA, respectively, and purified by centrifugation, and agarose gel electrophoresis analysis was performed to analyze whether there was siRNA-avidin on the adiposome surface. Lane 1 shows free siRNA-avidin; lane 2 shows siRNA-avidin with 0% Bio-PE adiposomes; lane 3 shows siRNA-avidin with 10% Bio-PE adiposomes; lane 4 shows siRNA-avidin with 20% Bio-PE adiposomes.

### DETAILED DESCRIPTION

### Example 1 Construction of biotin-labeled adiposomes

Materials: Phospholipids without biotin (DOPC), named 18:1 (Δ9-Cis) PC; biotin-modified phospholipid (Bio-PE), named 18:1 Biotinyl Cap PE; neutral lipid was glyceryl trioleate (TAG), all of the above products were purchased from Sigma.
(1) The above DOPC and Bio-PE were dissolved in chloroform at a final concentration of 25 mg/ml each.
(2) DOPC and Bio-PE were added to microcentrifuge tubes according to the following volumes, with mass ratios of Bio-PE of 0%, 1.25%, 5%, 10%, and 20%, respectively, and mixed by vortex; and the solvent were evaporated with high-purity nitrogen gas.

**Table 1**

| Sample | DOPC/µl | Bio-PE/µl | Bio-PE (m%) |
|---|---|---|---|
| 1 | 80 | 0 | 0 |
| 2 | 79 | 1 | 1.25 |
| 3 | 76 | 4 | 5 |
| 4 | 72 | 8 | 10 |
| 5 | 64 | 16 | 20 |

(3) 100 µL of phosphate buffer (PBS) and 5 mg of triglyceride (TAG) or other neutral lipids were added to the microcentrifuge tube, vortexed for 5 min (vortexing for 10 s and pausing for 5 s) to obtain a milky-white lipid mixture 1 (i.e., the initial preparation component), and the lipid mixture 1 was centrifuged at 1000 g for 5 min to remove the upper white band (FIG. 2A).
(4) The lipid mixture 1 was centrifuged at 20000 g for 5 min. After the centrifugation, the pellet at the bottom of the tube was removed (FIG. 2B), and the remaining emulsion in the tube was resuspended to obtain a milky-white lipid mixture 2.
(5) The lipid mixture 2 was centrifuged at 1000 g for 5 min. After the centrifugation, the upper white band was removed, and the remaining emulsion in the tube was resuspended to obtain a milky-white lipid mixture 3, which was the final biotin-labeled adiposome (FIG. 2C).

Further, the average particle size and PDI (polydispersity index) of adiposomes containing different amounts of biotin were detected using a dynamic light scattering instrument. The detection results of the prepared biotin-labeled adiposomes after being stored at 4°C for 1 day are shown in the following table:

**Table 2**

| Sample | Bio-PE (m%) | Size (nm) | PDI |
|---|---|---|---|
| 1 | 0 | 117.3±10.4 | 0.16±0.01 |
| 2 | 1.25 | 110.0±9.1 | 0.11±0.02 |
| 3 | 5 | 83.17±3.8 | 0.14±0.04 |
| 4 | 10 | 78.1±3.1 | 0.15±0.02 |
| 5 | 20 | 75.6±1.9 | 0.15±0.01 |

The results showed that when the amount of Bio-PE was 1.25% to 20%, as the content of biotin-PE in adiposomes increased, the average particle size gradually decreased, but the PDI remained within 0.2, indicating that the biotin-labeled adiposomes constructed by the present disclosure had high purity and good uniformity.

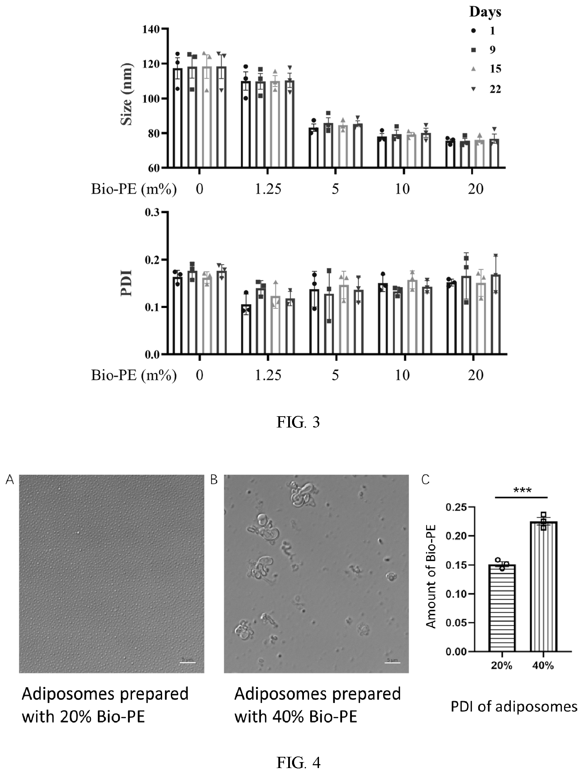

Adiposomes containing different amounts of biotin were stored at 4°C for 1 day, 9 days, 15 days, and 22 days, and changes in particle size and PDI were detected by dynamic light scattering. The results showed that the particle size of adiposomes containing different amounts of biotin remained unchanged, and the PDI also remained basically unchanged, indicating that they had good stability and uniformity (FIG. 3).

In addition, biotin-labeled adiposomes were prepared using 20% Bio-PE and 40% Bio-PE according to the above method, stored at 4°C for 1 day and detected, and the PDI and light microscopy results are shown in FIG. 4. The results showed that when the amount of Bio-PE added was 20%, the average particle size reached 75.6 nm, while when Bio-PE was added to 40%, optical microscopy showed many membrane impurities with non-adiposome-like structures (FIGs. 4-A and 4-B), and the PDI was greater than 0.2 (FIG. 4-C), significantly exceeding that of 20% Bio-PE-labeled adiposomes. PDI refers to polydispersity index, which is an indicator used to measure the uniformity of nanoparticles; the smaller the PDI, the more uniform the particle size distribution. When PDI is less than 0.2, it is generally considered to have good uniformity, while when PDI is greater than 0.2, the uniformity of nanoparticles is relatively poor.

According to the above preparation method, when the amount of added Bio-PE was 40%, the prepared adiposomes had more membrane impurities and the PDI was greater than 0.2. Therefore, the purification method was further optimized. When the amount of Bio-PE exceeded 20%, to obtain biotin-labeled adiposomes with higher purity and better uniformity, a separation and purification step was added in the subsequent operations of step 5 of Example 1, specifically: the lipid mixture 3 was centrifuged at 21000 g for 6 min. After the centrifugation, the pellet at the bottom of the tube was removed, and the remaining emulsion in the tube was resuspended to obtain a milky-white lipid mixture. The lipid mixture was centrifuged at 1000 g for 6 min. After the centrifugation, the upper white band was removed, and the remaining emulsion in the tube was resuspended. The above steps were repeated for a total of three times, and final adiposomes with different contents of biotin were obtained. According to the above steps, DOPC (25 mg/mL) and Bio-PE (25 mg/mL) prepared in the previous step (1) were added to microcentrifuge tubes according to the following volumes, respectively. The mass ratio of Bio-PE added was 0% to 100%, the sample numbers were 1 to 8, respectively, and adiposomes labeled with different contents of biotin were prepared.

**Table 3**

| Sample | DOPC/µl | Bio-PE/µl | Bio-PE (m%) |
|---|---|---|---|
| 1 | 80 | 0 | 0 |
| 2 | 79 | 1 | 1.25 |
| 3 | 76 | 4 | 5 |
| 4 | 72 | 8 | 10 |
| 5 | 64 | 16 | 20 |
| 6 | 48 | 32 | 40 |
| 7 | 16 | 64 | 80 |
| 8 | 0 | 80 | 100 |

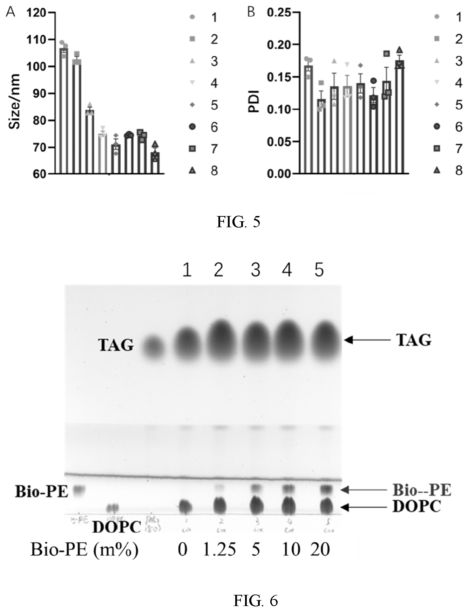

The average particle size and PDI (polydispersity index) of the above adiposomes with different contents of Bio-PE added were measured using a dynamic light scattering. The measurement results of the prepared biotin-labeled adiposomes after being stored at 4°C for 1 day are shown in Table 4 and FIG. 5.

**Table 4**

| Sample | Bio-PE (m%) | Size (nm) | PDI |
|---|---|---|---|
| 1 | 0 | 106.77±2.65 | 0.17±0.02 |
| 2 | 1.25 | 102.57±1.85 | 0.12±0.02 |
| 3 | 5 | 83.83±1.88 | 0.14±0.04 |
| 4 | 10 | 75.07±1.7 | 0.14±0.03 |
| 5 | 20 | 71.1±3.4 | 0.14±0.03 |
| 6 | 40 | 74.53±0.32 | 0.12±0.02 |
| 7 | 80 | 74.27±1.46 | 0.14±0.04 |
| 8 | 100 | 68.13±2.87 | 0.18±0.01 |

The results showed that using the optimized method, the results of the first 5 samples were consistent with those without optimization; as the content of biotin-PE in adiposomes increased, the average particle size gradually decreased, and the PDI remained within 0.2. However, when the amount of added Bio-PE exceeded 20% and reached 40%, the purity and uniformity were improved, and the PDI was also below 0.2. Even when the amount of Bio-PE added reached 100%, the PDI was also below 0.2. In addition, as the Bio-PE content increased, the final particle size of biotin-labeled adiposomes gradually decreased, eventually stabilizing at about 70 nm. The above results indicate that through the optimized purification method, when the mass ratio of Bio-PE added is 0% to 100%, the prepared adiposomes labeled with different contents of biotin have high purity and good uniformity.

### Example 2 Detection of lipid composition of biotin-labeled adiposomes

The lipid composition of adiposomes containing different amounts of biotin was detected and analyzed by TLC (thin-layer chromatography).

The above-constructed adiposomes containing different amounts of biotin were added with an equal volume of methanol and 2 times the volume of chloroform to extract lipids. The organic phase was collected and dried with nitrogen to obtain total lipids. The obtained total lipids were added to 100 µL of chloroform, and 10 µL was loaded onto a silica gel plate and developed in a developer of n-hexane:diethyl ether:glacial acetic acid (volume ratio of 80:20:1) to separate TAG. Then the silica gel plate was placed in a solvent of chloroform:methanol:glacial acetic acid:water (volume ratio of 75:13:9:3) for development to separate DOPC and Bio-PE.

The results showed that, corresponding to the raw materials added during the preparation of adiposomes, the lipid compositions of the 5 groups of adiposome samples were similar. The neutral lipids were all TAG, and the phospholipids were mainly DOPC. In sample No. 1, since no Bio-PE was added, the Bio-PE signal could not be detected in adiposomes, while in samples No. 2 to 5, since 1.25%, 5%, 10%, and 20% Bio-PE were added respectively, the Bio-PE signal could be detected, and as the amount of added Bio-PE increased, the signal of biotin on adiposomes also became stronger (as indicated by arrows in FIG. 6).

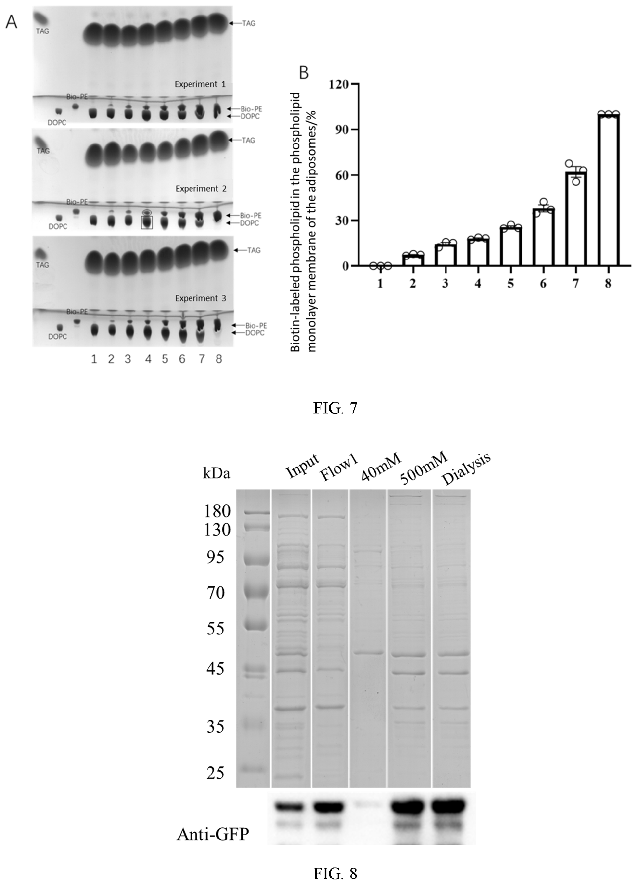

Similarly, the lipid composition of adiposomes labeled with different contents of biotin prepared by the optimized purification method with the mass ratio of added Bio-PE of 0% to 100% was further detected, and the content of biotin-labeled phospholipids in the adiposome phospholipid monolayer. The results are shown in FIG. 7.

The results showed that, corresponding to the raw materials added during the preparation of adiposomes, the lipid compositions of the 8 groups of adiposome samples were similar. The neutral lipids were all TAG, and the phospholipids were mainly DOPC. In sample No. 1, since no Bio-PE was added, the Bio-PE signal could not be detected in adiposomes, while in samples No. 2 to 8, since 1.25% to 100% Bio-PE was added respectively, the Bio-PE signal could be detected, and as the amount of added Bio-PE increased, the signal of biotin on adiposomes also became stronger (FIG. 7-A). Further, the grayscale value of each sample on the silica gel plate was analyzed by software Image J to calculate the content proportion of biotin-labeled phospholipids in the adiposome phospholipid monolayer. Taking sample No. 6 of experiment 1 as an example, the point in the circle is the Bio-PE signal, and the point in the square is the DOPC signal. The content proportion of biotin-labeled phospholipids in the adiposome phospholipid monolayer was calculated by the formula: grayscale value of the point in the circle/(grayscale value of the point in the circle + grayscale value of the point in the square), as shown in FIG. 7-B. The calculated Bio-PE content in adiposomes of 8 samples in three independent experiments is shown in the following table:

**Table 6:**

| Sample No. | Bio-PE feed amount/m% | Bio-PE content in adiposome phospholipid monolayer/% | | | Mean±SD |
|---|---|---|---|---|---|
| | | Experiment 1 | Experiment 2 | Experiment 3 | |
| No. 1 | 0 | 0 | 0 | 0 | 0±0 |
| No. 2 | 1.25 | 6.61 | 7.31 | 7.80 | 7.24±0.6 |
| No. 3 | 5 | 15.73 | 15.16 | 12.27 | 14.39±1.86 |
| No. 4 | 10 | 18.20 | 18.66 | 17.28 | 18.05±0.7 |
| No. 5 | 20 | 27.24 | 24.72 | 25.17 | 25.71±1.34 |
| No. 6 | 40 | 42.34 | 37.12 | 34.70 | 38.05±3.9 |
| No. 7 | 80 | 67.73 | 63.18 | 55.68 | 62.2±6.08 |
| No. 8 | 100 | 100 | 100 | 100 | 100±0 |

The above results indicated that when the mass ratio of Bio-PE added was 0% to 100%, as the mass ratio of Bio-PE added increased, the content proportion of biotin-labeled phospholipids in the phospholipid monolayer of the formed adiposomes also gradually increased, with calculated results of 0% to 100%. This indicated that biotin-labeled adiposomes with high purity and good uniformity were successfully constructed.

### Example 3 Construction of avidin-tagged recombinant protein GFP-avidin

Materials: E. coli Rosetta was a product from TIANGEN; nickel-chelating Chelating Sepharose Fast Flow was a product from GE Healthcare, and the column was a product from Thermo. Buffer A formulation: 500 mM Tris, 1.5 M sodium chloride, 40% glycerol (v/v), with hydrochloric acid added to adjust pH to 7.8.

The gene sequence of the target protein GFP-avidin was inserted into the expression vector pET28a(+), designated as pET28a-GFP-avidin, and the vector was transformed into Rosetta competent cells, and single colonies were picked. The Rosetta cells containing pET28a-GFP-avidin were inoculated into LB liquid medium containing kanamycin and cultured at 37°C with shaking at 200 rpm. When the bacterial concentration reached OD600=0.6, isopropylthiogalactoside (IPTG) was added to the culture system to a final concentration of 0.4 mM, and then the culture system was induced at 16°C for 24 hours. The above-cultured bacteria were then disrupted using a high-pressure cell disruptor, and the resulting bacterial lysate was subjected to ultracentrifugation at 30000 g for 60 min to collect the supernatant. 50 µL of the supernatant was taken and mixed with an equal volume of 2x Sample Buffer, and the resulting mixture was used as Sample 1 (Input). The remaining supernatant was incubated with the nickel-chelating packing Chelating Sepharose Fast Flow, and after incubation at 4°C for 2 hours, the mixture was transferred to a 4 mL column to collect the flow-through liquid. 50 µL of the flow-through liquid was taken and mixed with an equal volume of 2x Sample Buffer, and the resulting mixture was used as Sample 2 (Flow1). The packing in the column was resuspended with 40 mM imidazole to wash non-specific bands, and the washing liquid was collected. 50 µL of the washing liquid was taken and mixed with an equal volume of 2x Sample Buffer, and the resulting mixture was used as Sample 3 (40 mM). The target protein was then eluted with 500 mM imidazole, and the flowing elution liquid was collected. 50 µL of the elution liquid was taken and mixed with an equal volume of 2x Sample Buffer, and the resulting mixture was used as Sample 4 (500 mM). The resulting mixture was placed in a dialysis bag, and the dialysis bag was placed in a beaker containing Buffer A at 100 times the volume of the dialysis bag for overnight dialysis. 50 µL of the solution in the dialysis bag was taken and mixed with an equal volume of 2x Sample Buffer, and the resulting mixture was used as Sample 5 (Dialysis). The above samples were analyzed by SDS-PAGE, followed by Coomassie staining analysis and Western blot (WB) identification.

Coomassie staining results showed that there was a distinct protein band at the position of 45 kDa, with a size consistent with the molecular weight of the GFP-avidin sequence. Meanwhile, WB results also showed a distinct band, indicating that the avidin-tagged recombinant protein GFP-avidin were successfully constructed (FIG. 8).

### Example 4 Biotin-labeled adiposomes carrying target protein GFP

50 µL of adiposomes prepared in Example 1 with 0%, 1.25%, 5%, 10%, and 20% Bio-PE added (OD600=2.0) were respectively added into EP tubes, and then 20 µL (1.0 mg/mL) of the avidin-tagged recombinant protein GFP-avidin prepared in Example 3 was added. After mixing by vortex, the mixture was incubated at room temperature for 2 hours, with gentle tapping for mixing every 10 minutes. After 2 hours, the mixture was centrifuged at 20000 g for 5 min. After the centrifugation, the pellet at the bottom of the tube and the lower emulsion layer were removed, leaving only the upper white band portion. Phosphate buffer was added to resuspend the white band portion, followed by centrifugation at 20000 g for 5 min, and the lower emulsion layer was removed, leaving only the upper white band portion. The above steps were repeated twice, and the finally obtained resuspended white band portion was non-biotin-labeled or biotin-labeled adiposomes without or with GFP-avidin. The adiposomes were identified by confocal laser scanning microscopy and silver staining to analyze whether the recombinant protein GFP-avidin could be bound to non-biotin-labeled or biotin-labeled adiposomes (FIG. 9).

FIG. 9A showed the results of adiposomes with 0% Bio-PE (FIG. 9-A-b) and the recombinant protein GFP-avidin (FIG. 9-A-a) after incubation and centrifugation purification. Since no Bio-PE was added during the preparation of adiposomes, there was no biotin on the adiposomes. The results showed that the recombinant protein GFP-avidin could not bind to adiposomes without biotin (no green signal was observed in FIG. 9-A-c).

In FIGs. 9B to E, since different amounts of Bio-PE were added during the preparation of adiposomes, the adiposomes carried different amounts of biotin. The results showed that the recombinant protein GFP-avidin could bind to biotin-labeled adiposomes (there were green ring-like signals on the red spherical surfaces in FIGs. 9-B-c, 9-C-c, 9-D-c, and 9-E-c, as indicated by white arrows). Among them, adiposomes prepared with 10% to 20% Bio-PE added were better for recruiting the target protein GFP-avidin, where the formed ring-like structures were more uniform and the morphology of adiposomes was more complete (FIG. 9E). However, for adiposomes prepared with other amounts of Bio-PE (1.25% to 5%), GFP-avidin was mostly in a punctate structure, which affected the morphology of adiposomes and caused aggregation of adiposomes (FIGs. 9B-9C), with decreased capability of recruiting protein. The results indicated that adiposomes labeled with different amounts of biotin affected the quantity and shape of proteins recruited, and also affected the morphology and stability of adiposomes.

Consistent with the results of confocal laser scanning microscopy, silver staining results (FIG. 10) showed that biotin-labeled adiposomes could successfully bind to the recombinant protein GFP-avidin.

### Example 5 Biotin-labeled adiposomes carrying protein horseradish peroxidase (HRP)

Horseradish peroxidase is commonly used to catalyze ECL-type reagents to produce chemiluminescence in Western experiments and to catalyze TMB to produce blue color in ELISA experiments. In order to better prove the wide application of the biotin-labeled adiposome nanocarrier system provided by the present disclosure in carrying protein-based substances, biotin-labeled adiposomes were used to carry the protein horseradish peroxidase (HRP) and whether the HRP carried by adiposomes had biological activity was further investigated.

Since different amounts of Bio-PE affected the purity and uniformity of adiposomes, adiposomes of the above 8 samples were prepared according to the optimized method in Example 1, wherein adiposomes with 0% Bio-PE content served as a negative control. 50 µL of the above prepared adiposomes (OD600=4.0) were respectively added into EP tubes, and then 10 µL of avidin-labeled horseradish peroxidase (HRP-avidin, purchased from Beyotime) was respectively added. After mixing by vortex, the mixture was incubated at room temperature for 2 hours, with gentle tapping for mixing every 10 minutes. After 2 hours, the mixture was centrifuged at 20000 g for 5 min. After centrifugation, the pellet at the bottom of the tube and the lower emulsion layer were removed, leaving only the upper white band portion. Phosphate buffer was added to resuspend the white band portion, followed by centrifugation at 20000 g for 5 min, and the lower emulsion layer was removed, leaving only the upper white band portion. The above steps were repeated twice, and the finally obtained resuspended white band portion was adiposomes without or with HRP-avidin. The activity of horseradish peroxidase on adiposomes was detected by TMB (3,3',5,5'-Tetramethylbenzidine), and the absorbance at 450 nm of different samples was detected by a microplate reader. The results showed that as the content proportion of biotin-labeled phospholipids in the phospholipid monolayer of adiposomes gradually increased, the intensity of absorbance also gradually increased (see FIG. 11). The above results indicated that biotin-labeled adiposomes are also capable of carrying avidin-labeled horseradish peroxidase (HRP), and the HRP carried by adiposomes had biological activity.

### Example 6 Biotin-labeled adiposomes carrying small-molecule fluorescein isothiocyanate (FITC)

Fluorescein isothiocyanate (FITC) is commonly used in biology to detect or track the interaction of conjugates with other biological molecules, with a molecular weight of 389 g/mol. In order to better prove the wide application of the biotin-labeled adiposome nanocarrier system provided by the present disclosure in carrying small-molecule substances, biotin-labeled adiposomes were used to carry fluorescein isothiocyanate (FITC).

Since different amounts of Bio-PE affected the purity and uniformity of adiposomes, adiposomes with 1.25% and 40% Bio-PE were prepared according to the optimized method in Example 1, and adiposomes with 0% Bio-PE were also prepared as a negative control. 50 µL of the above prepared adiposomes (OD600=4.0) was respectively added into EP tubes, and then 10 µL of avidin-labeled fluorescein isothiocyanate (FITC) (FITC-avidin, purchased from Solarbio) was respectively added. After mixing by vortex, the mixture was incubated at room temperature for 2 hours, with gentle tapping for mixing every 10 minutes. After 2 hours, the mixture was centrifuged at 20000 g for 5 min. After the centrifugation, the pellet at the bottom of the tube and the lower emulsion layer were removed, leaving only the upper white band portion. Phosphate buffer was added to resuspend the white band portion, followed by centrifugation at 20000 g for 5 min, and the lower emulsion layer was removed, leaving only the upper white band portion. The above steps were repeated twice, and the finally obtained resuspended white band portion was adiposomes without or with FITC-avidin. Analysis by confocal laser scanning microscopy revealed that the signals of adiposomes prepared with 1.25% and 40% Bio-PE (12a) and the signals of FITC-avidin (12b) overlapped (12c), while no FITC-avidin signal was detected on the surface of adiposomes with 0% Bio-PE (see FIG. 12A). The above results indicated that biotin-labeled adiposomes are capable of carrying small-molecule fluorescein isothiocyanate (FITC).

### Example 7 Biotin-labeled adiposomes carrying nucleic acid

Nucleic acid substances mainly include two major categories, namely deoxyribonucleic acid (DNA) and ribonucleic acid (RNA), which have many important applications in the medical field, such as pathogen detection, gene therapy, and vaccines. In order to better prove the wide application of the biotin-labeled adiposome nanocarrier system provided by the present disclosure in carrying nucleic acid substances, biotin-labeled adiposomes were used to carry siRNA (small interfering RNA).

Since different contents of Bio-PE affect the purity and uniformity of adiposomes, adiposomes with 10% and 20% Bio-PE were prepared according to the method of Example 1, and adiposomes with 0% Bio-PE were prepared as a control. 50 µL of each of the above-prepared adiposomes (OD600=4.0) was added into EP tubes, and then 20 µL of avidin-labeled siRNA (synthesized by Tsingke Biotech Co., Ltd., used to reduce GFP protein expression in cells/tissues, with the sequence SEQ ID NO: 3: 5'-GACGUAAACGGCCACAAGUTT-3') was added respectively. After mixing by vortex, the mixture was incubated at room temperature for 2 hours, with gentle tapping for mixing every 10 minutes. After 2 hours, the mixture was centrifuged at 20000 g for 5 min. After the centrifugation, the precipitate at the bottom of the tube and the lower emulsion layer were removed, leaving only the upper white band portion. Phosphate buffer was added to resuspend the white band portion, followed by centrifugation at 20000 g for 5 min, removing the lower emulsion layer and leaving only the upper white band portion. The above steps were repeated twice, and the finally obtained resuspended white band portion was adiposomes without or with siRNA-avidin. Agarose gel electrophoresis analysis (see FIG. 13) revealed that adiposomes prepared with 10% and 20% Bio-PE could carry avidin-labeled siRNA, and the siRNA signal of free siRNA-avidin was enriched in the loading well of the agarose gel without migration. Adiposomes prepared with 0% Bio-PE could not carry avidin-labeled siRNA, and siRNA-avidin showed significant migration in the agarose gel. The above results indicate that biotin-labeled adiposomes are capable of carrying nucleic acid substances.

The foregoing are merely preferred embodiments of the present disclosure. It should be noted that, for a person of ordinary skill in the art, several changes and modifications can be made without departing from the principle of the present disclosure, and these changes and modifications should also be considered as within the scope of protection of the present disclosure.

## Claims

1. A method for producing a biotin- or avidin-labeled adiposome, comprising:
mixing biotin- or avidin-labeled phospholipid, unlabeled phospholipid, and neutral lipid in a solvent, and performing reaction to obtain the biotin- or avidin-labeled adiposome.

2. The method according to claim 1, wherein a mass percentage of the biotin- or avidin-labeled phospholipid in the total phospholipids is X, and X is any range selected from the group consisting of 0 < X < 40%, 40% ≤ X ≤ 100%, 0 < X < 1.25%, 1.25% ≤ X ≤ 5%, 5% < X < 10%, 10% ≤ X ≤ 20%, 20% < X < 40%, 40% ≤ X < 60%, 60% ≤ X < 80%, and 80% ≤ X ≤ 100%.

3. The method according to claim 1, wherein prior to mixing, the method further comprises: dissolving the unlabeled phospholipid and the biotin- or avidin-labeled phospholipid in chloroform, respectively.

4. The method according to claim 1, wherein the neutral lipid is at least one selected from the group consisting of triglyceride, cholesteryl ester, retinyl ester, ether ester, sterol ester, and polyhydroxyalkanoate; and the phospholipid is at least one selected from the group consisting of phosphatidylcholine (PC), phosphatidylethanolamine (PE), phosphatidic acid (PA), phosphatidylserine (PS), PE, and phosphatidylinositol (PI).

5. The method according to claim 1, wherein a mass ratio of the unlabeled phospholipid, the biotin- or avidin-labeled phospholipid, and the neutral lipid is (0 to 2):(0 to 2):(3 to 10), preferably (0 to 2):(0 to 2):5.

6. The method according to claim 5, wherein the mass ratio of the unlabeled phospholipid, the biotin- or avidin-labeled phospholipid, and the neutral lipid is (1.975 to 2):(0 to 0.025):5, (1.2 to 1.975):(0.025 to 0.8):5, (1.6 to 1.975):(0.025 to 0.4):5, (1.6 to 1.8):(0.2 to 0.4):0.5, (1.2 to 1.6):(0.4 to 0.6):5, (0 to 1.2):(0.8 to 2):5, (0.4 to 1.2):(0.8 to 1.6):5, or (0 to 0.4):(1.6 to 2):5.

7. The method according to claim 1, wherein the solvent is at least one selected from the group consisting of phosphate buffer, HEPES buffer, sucrose solution, NaCl solution, KCl solution, and MgCl₂ solution.

8. The method according to claim 1, wherein the mixing includes vortex mixing, ultrasonic mixing, stirring mixing, and high-pressure homogenization mixing.

9. The method according to claim 8, wherein the vortex mixing is performed by: vortexing at 3000 to 4000 rpm, vortexing for 8 to 15 s and pausing for 5 to 15 s each time, for a total duration of 3 to 7 min.

10. The method according to claim 1, wherein after the reaction is completed, the method further comprises steps of centrifuging and collecting a liquid phase.

11. The method according to claim 10, wherein the centrifuging and collecting the liquid phase specifically comprises:
1) subjecting the vortexed solution to a first centrifugation at 100 g to 1000 g for 3 to 7 min, and removing an upper white band; performing a second centrifugation at 18000 g to 22000 g for 3 to 7 min, removing a precipitate, and collecting a liquid-phase emulsion;
2) resuspending the liquid-phase emulsion, performing a third centrifugation at 100 g to 1000 g for 3 to 7 min, removing an upper white band, and collecting a liquid-phase emulsion to obtain a biotin-labeled adiposome.

12. The method according to claim 10 or 11, wherein after the centrifuging and collecting the liquid phase, the method further comprises a step of purification, wherein the purification comprises:
first centrifuging at 15000 g to 2300 g for 5 to 10 min, removing a precipitate, and resuspending; then centrifuging at 500 g to 1500 g for 5 to 10 min, removing an upper white band, and resuspending; repeating this step 3 times.

13. The method according to claim 11 or 12, wherein a solution used for the resuspending is at least one selected from the group consisting of phosphate buffer, HEPES buffer, sucrose solution, NaCl solution, KCl solution, and MgCl₂ solution.

14. A biotin- or avidin-labeled adiposome produced by the method according to any one of claims 1 to 13.

15. A biotin- or avidin-labeled adiposome, wherein a phospholipid monolayer of the adiposome comprises biotin- or avidin-labeled phospholipid; and
in the phospholipid monolayer membrane of the adiposome, an average percentage of the content of the biotin- or avidin-labeled phospholipid is Y, and 0 < Y ≤ 100%, preferably 5 ≤ Y ≤ 100%.

16. The biotin- or avidin-labeled adiposome according to claim 14, wherein Y is selected from the group consisting of 5% to 20%, 10% to 20%, 15% to 30%, 20% to 40%, 35% to 65%, and 60% to 100%.

17. The biotin- or avidin-labeled adiposome according to any one of claims 14 to 16, wherein an average particle size of the biotin- or avidin-labeled adiposome is 60 nm to 150 nm, preferably 65 nm to 120 nm.

18. The biotin- or avidin-labeled adiposome according to claim 17, wherein the average particle size of the biotin- or avidin-labeled adiposome is 60 nm to 75 nm, 70 nm to 75 nm, 75 nm to 85 nm, 80 nm to 105 nm, 100 nm to 110 nm, 105 nm to 115 nm, 110 nm to 120 nm, or 120 nm to 150 nm.

19. The biotin- or avidin-labeled adiposome according to any one of claims 15 to 18, wherein a method for producing the biotin- or avidin-labeled adiposome comprises:
mixing biotin- or avidin-labeled phospholipid, unlabeled phospholipid, and neutral lipid in a solvent, and performing reaction to obtain the biotin- or avidin-labeled adiposome; or
producing an adiposome, adding a lipid droplet resident protein carrying avidin or biotinlabeling, incubating, and obtaining an avidin-labeled adiposome.

20. Use of a biotin- or avidin-labeled adiposome produced by the method according to any one of claims 1 to 13, or the biotin- or avidin-labeled adiposome according to any one of claims 14 to 19, as a nanocarrier in the manufacture of a medicament.

21. A nanocarrier system, comprising:
a biotin-labeled adiposome and an avidin-labeled cargo of interest; or
an avidin-labeled adiposome and a biotin-labeled cargo of interest;
wherein the adiposome and the cargo of interest are connected non-covalently via biotin and avidin.

22. The nanocarrier system according to claim 21, wherein the biotin- or avidin-labeled adiposome is produced by the method according to any one of claims 1 to 13, or is the biotin- or avidin-labeled adiposome according to any one of claims 14 to 19.

23. The nanocarrier system according to claim 21, wherein the cargo of interest is a pharmaceutically active ingredient, and the pharmaceutically active ingredient includes a peptide, an antibody, a nucleic acid, or a small molecule.

24. The nanocarrier system according to any one of claims 21 to 23, wherein the cargo of interest is a marker protein, and the marker protein includes GFP protein, mCherry protein, Luciferase protein, and/or horseradish peroxidase.

25. The nanocarrier system according to any one of claims 21 to 23, wherein the cargo of interest is a nucleic acid, preferably DNA or RNA, and more preferably one or more selected from the group consisting of mRNA, RNAi, siRNA, shRNA, miRNA, sgRNA, and crRNA.
